# EUROPEAN PATENT SPECIFICATION

(11) **EP 2 177 110 B1**
(45) Date of publication and mention of the grant of the patent: **12.11.2014**
(21) Application number: 09172192.8
(22) Date of filing: 05.10.2009
(51) Int. Cl.: A23L 1/00, A23L 1/03, A23L 1/187, A23L 1/19, A23L 1/236, A23L 1/30, A61K 35/74

(54) **Immunostimulating composition containing lactic acid bacteria**
Immunstimulierende Zusammensetzung mit Milchsäurebakterien
Composition immunostimulante contenant des bactéries de l'acide lactique

(30) Priority: 16.10.2008 JP 2008267037; 20.10.2008 US 136991 P
(43) Date of publication of application: 21.04.2010
(73) Proprietor: House Wellness Foods Corporation, Hyogo 664-0011 (JP)
(72) Inventor: Fujiki, Takashi, Itami-shi Hyogo 664-0846 (JP); Kawasaki, Kengo, Itami-shi Hyogo 664-0893 (JP); Hirose, Yoshitaka, Itami-shi Hyogo 664-0001 (JP); Murosaki, Shinji, Nara-shi Nara 630-8114 (JP); Yamamoto, Yoshihiro, Itami-shi Hyogo 664-0002 (JP)
(74) Representative: Kalhammer, Georg

(56) References cited:
- EP-A- 2 042 590
- WO-A-03/033681
- JP-A- 2001 333 723
- DATABASE WPI Week 200537 Thomson Scientific, London, GB; AN 2005-359468 XP002558863 & JP 2005 124502 A (KABU KK) 19 May 2005 (2005-05-19)

## Description

### BACKGROUND OF THE INVENTION

### Field of the invention

The present invention relates to a method for producing an immunostimulating composition containing Lactobacillus plantarum L-137 having IL-12 production-inducing activity.

### Description of related art

Lactic acid bacteria belonging to the genus Lactobacillus are known to activate macrophages to promote production of IL-12 (interleukin 12), which is a cytokine that activates natural killer cells. Lactic acid bacteria belonging to the genus Lactobacillus are immunostimulators without any substantial side effects, and therefore, are suitable for regular use and also effective when used in combination with other immunostimulators (JP-A No. 10-167972).

Products containing lactic acid bacteria belonging to the genus Lactobacillus as an immunostimulator have conventionally been proposed. For example, JP-A No. 11-228425 describes an IL-12 production-inducing composition containing bacteria belonging to the genus Lactobacillus or processed product thereof, and a saccharide having 3-O-α-D-glucopyranosyl D-glucose as a structural unit. JP-A No. 2002-80364 describes a preparation containing ascorbic acid and lactic acid bacteria belonging to the genus Lactobacillus as an immunostimulator. JP-A No. 2007-204488 describes an immunoenhancing composition containing vitamin E and lactic acid bacteria belonging to the genus Lactobacillus.

When lactic acid bacteria are added to a medicinal or food product, killed cells are usually used since they are easier to handle than viable cells. However, depending on at which step in a culture process lactic acid bacteria are killed, the IL-12 production-inducing activity of obtained killed cells varies considerably. Consequently, there has been an expectation of completion of a method for preparing killed cells by which method killed cells that keep high IL-12 production-inducing activity can always be obtained.

EP 2 042 590 discloses an agent for enhancing growth and improving viability of a lactic acid bacterium comprising a dead cell of a lactic acid bacterium as an active ingredient.

JP 2001-333723 A describes a method for producing frozen bean curds by adding a coagulant, dried and dead lactobacillus coccus and modified starch with a soybean milk, dissolving and dispersing, then heating for making it uniform, filling in a container for solidifying and then freezing quickly.

JP 2005-124502 A discloses the preparation of processed food containing lactic acid bacteria product comprising culturing lactic acid bacteria, sterilizing the culture to produce lactic acid bacteria product comprising dead lactic acid bacteria, adding the product to raw material of processed food and processing the raw material.

WO 03/033681 A2 discloses a probiotic strain termed Lactobacillus pentosus NCIMB 41114, capable of preventing growth of pathogenic microorganism in the gastrointestinal tract.

Murosaki et al. (1998) J. Allergy Clin. Immunol. 102, 57-64 describes experiments using heat-killed cells of Lactobacillus plantarum L-137 as IL-12 production-inducing agents. Murosaki et al. suggest that L. plantarum L-137 is useful for prevention and treatment of food allergy.

### SUMMARY OF THE INVENTION

### Technical problem

The main object of the present invention is to provide a method for producing an immunostimulating composition containing lactic acid bacteria, the composition effectively inducing IL-12 production in the body, the method comprising preparing killed cells of lactic acid bacteria in such a manner that obtained killed cells keep high IL-12 production-inducing activity and adding the killed cells obtained without loss of its activity to a food-or-drink product.

### Solution to problem

The present invention provides the following method for producing an immunostimulating composition:
(1) A method for producing an immunostimulating composition containing lactic acid bacteria having IL-12 production-inducing activity, comprising culturing the lactic acid bacteria and then killing the lactic acid bacteria within 30 minutes from the point of time at which the decrease of the pH of the culture medium gets less than 0.1 per 3 hours, and adding the killed cells of the lactic acid bacteria to a food-or-drink product, wherein the lactic acid bacteria are Lactobacillus plantarum L-137.
(2) The method according to (1), wherein the lactic acid bacteria are killed by heat.
(3) The method of (1) or (2), wherein the food-or-drink product is a drink.
(4) The method according to (3), wherein the drink is selected from the group consisting of energy drink, sports supplement drink, isotonic drink, functional water, bottled water, flavored water, flavored drink, milk-free meal-substitute drink, fruit juice, fruit drink, vegetable juice, coffee drink, tea drink, green tea drink, oolong tea drink and blended tea-based drink.
(5) The method of (1) or (2), wherein the food-or-drink product is a powdered food.
(6) The method according to (5), wherein the powdered food is selected from the group consisting of powdered drink, powdered energy drink, powdered sports supplement drink, powdered isotonic drink, powdered functional water, powdered bottled water, powdered flavored water, powdered flavored drink, powdered cocoa, powdered malt drink, powdered soup, tabletop artificial sweetener, powdered tea, powdered green tea, powdered plum tea, powdered seaweed tea, powdered juice and instant coffee.

### Advantageous effects of invention

According to the present invention, a method for producing an immunostimulating composition containing Lactobacillus plantarum L-137, the method comprising preparing killed cells of Lactobacillus plantarum L-137 that keep high IL-12 production-inducing activity and adding the killed cells obtained without loss of its activity to a food-or-drink product, can be provided. By regularly taking the above-mentioned immunostimulating composition containing Lactobacillus plantarum L-137, the induction of IL-12 production in the body is improved, immune activity is enhanced, and health promotion can be realized.

### DESCRIPTION OF THE PREFERRED EMBODIMENTS

Hereafter, the present invention will be described in detail.

### (I) Method for producing an immunostimulating composition containing Lactobacillus plantarum L-137

The method of the present disclosure for producing an immunostimulating composition containing Lactobacillus plantarum L-137 is a method comprising adding killed cells of Lactobacillus plantarum L-137 to a food-or-drink product.

### Lactobacillus plantarum L-137

Lactobacillus plantarum L-137 (FERM BP-08607; International Patent Organism Depository, National Institute of Advanced Industrial Science and Technology) is used in accordance with this invention.

Lactobacillus plantarum L-137 can be proliferated by culture on various culture media, such as a natural medium, a synthetic medium, and a semisynthetic medium. The culture medium contains a nitrogen source and a carbon source. The nitrogen source may be, for example, a meat extract, peptone, gluten, casein, a yeast extract, an amino acid, etc., and the carbon source may be, for example, glucose, maltose, xylose, fructose, inositol, starch syrup, koji extract, starch, bagasse, wheat bran, molasses, glycerol, etc. In addition, minerals such as ammonium sulfate, potassium phosphate, magnesium chloride, sodium chloride, iron, manganese and molybdenum; vitamins; etc. may be added. The culture temperature may be about 25 to 40°C, preferably about 27 to 35°C, and the culture duration may be about 12 to 48 hours, optionally with aerated shaking.

### Killed cells

Cultured Lactobacillus plantarum L-137 may be killed in culture media, or after separation from the culture by centrifugation etc. Examples of the killing method include, for example, heating, ultraviolet irradiation, formalin treatment, etc. Inter alia, heating is preferred because high IL-12 production inducing activity can be maintained. The temperature for killing lactic acid bacteria is preferably about 65 to 100°C, more preferably about 70 to 90°C, and more preferably about 75 to 85°C. The heating duration for killing lactic acid bacteria is preferably about 5 to 90 minutes, more preferably about 10 to 60 minutes, and more preferably about 15 to 30 minutes. Within the above-mentioned ranges, Lactobacillus plantarum L-137 can be killed while the decline of IL-12 production inducing activity is suppressed.

The killed cells of Lactobacillus plantarum L-137 can be used in paste form or in dried powder form. Use in powder form is preferred for ease of handling. The separated cells should not be further grinded, crushed, enzymatically decomposed, or extracted because such a process reduces the IL-12 production inducing activity.

### pH of culture medium

In the method of the present invention for producing an immunostimulating composition containing Lactobacillus plantarum L-137, used are killed cells obtained by culturing Lactobacillus plantarum L-137 and then immediately killing the bacteria at the point of time at which the pH of the culture medium substantially stops decreasing. The reason is that killed cells obtained in this way keep high IL-12 production-inducing activity.

"The point of time at which the pH of the culture medium substantially stops decreasing" means the point of time at which the decreasing rate of the pH of the culture medium, the pH being measured at regular time intervals, gets less than 0.1 per 3 hours. It is preferable to conduct a preliminary test in order to determine the criterion depending on the culture medium, the culture condition, etc. To give an actual example, in the case where Lactobacillus plantarum L-137 is cultured in 200 ml of modified GYP medium at 32°C, the pH of the culture medium substantially stops decreasing 18 hours after inoculation (see Examples).

Also, "immediately" means that Lactobacillus plantarum L-137 should be killed by starting heating within about 30 minutes from the point at which the pH of the culture medium substantially stops decreasing. A case where Lactobacillus plantarum L-137 is killed after being stored in a condition that prevents the bacteria from proliferating (for example, at a low temperature) is not included.

### Addition to a food-or-drink product

The killed cells may be mixed with other materials during production of a food-or-drink product, or added at the end of the production of a food-or-drink product.

The food-or-drink product means what is orally ingested, and examples thereof include a drink, a fermented food, a semisolid food, a solid food, a powdered food, etc.

Examples of the drink include energy drink, sports supplement drink, isotonic drink, functional water, bottled water, flavored water, flavored drink, milk-added meal-substitute drink, flavored milk, milk drink, milk-free meal-substitute drink, soy milk, fruit juice, nectar, fruit drink, vegetable juice, milk, coffee drink, tea drink, green tea drink, oolong tea drink, blended tea-based drink, etc.

Examples of the fermented food include yogurt, fermented milk, yogurt drink, etc.

Examples of the semisolid food include tofu, high density liquid diet, jelly drink, jelly, mousse, pudding, etc.

Examples of the solid food include instant breakfast cereal, cereal bar, energy bar, nutrition bar, soy bar, chocolate, low-fat spread, tofu hamburger steak, etc.

Examples of the powdered food include powdered drink, powdered energy drink, powdered sports supplement drink, powdered isotonic drink, powdered functional water, powdered bottled water, powdered flavored water, powdered flavored drink, powder cocoa, powdered malt drink, powdered soup, tabletop artificial sweetener, creaming powder, infant formula, pizza powder, takoyaki powder, okonomiyaki powder, pancake mix, skim milk, powdered tea, powdered green tea, powdered plum tea, powdered seaweed tea, powdered juice, instant coffee, etc.

### Amount of killed cells to be added

The amount of killed cells added to a food-or-drink product is preferably determined taking the absorption rate into consideration, in such a way that about 0.5 to 200 mg, more preferably about 1 to 100 mg, and more preferably about 2 to 50 mg of dried killed cells will be ingested daily by an adult weighing about 60 kg.

### (II) Immunostimulating composition containing Lactobacillus plantarum L-137

The immunostimulating composition containing Lactobacillus L-137 of the present disclosure is an immunostimulating composition containing Lactobacillus plantarum L-137 having IL-12 production inducing activity obtained by the above-described method.

Administration of the immunostimulating composition containing Lactobacillus plantarum L-137 of the present disclosure to a mammal or a bird induces IL-12 production in the body of the mammal or the bird. The immune system of a mammal or a bird to which the immunostimulating composition containing Lactobacillus plantarum L-137 of the present disclosure has been administered is activated, and thereby health promotion can be realized. The mammal may be a human, a mouse, a rat, a goat, a sheep, a pig, a cow, a horse, a dog, a cat, etc. The bird may be a pigeon, a sparrow, a duck, an ostrich, a quail, a turkey, a goose, etc. The route of administration is not limited, and usually the composition is administered orally.

### Examples

Hereinafter, the present invention will be described in more detail by Examples, but it is not limited thereto.

### Test example 1

### Comparison of IL-12 production inducing activity of various lactic acid bacteria

To 200 ml each of modified GYP medium, which is a culture medium for lactic acid bacteria, Bifidobacterium longum, Bifidobacterium bifidum, Lactobacillus casei, Lactobacillus delbrueckii, Lactobacillus acidophilus, Lactobacillus plantarum JCM1149, and Lactobacillus plantarum L-137 were separately inoculated in an amount of 1% by weight as a starter, and cultured at 32°C for 24 hours. After culture, sterilization at 80°C for 20 minutes and subsequent centrifugation at 3000 rpm for 20 minutes were performed. Then supernatant was removed for cell collection. The collected cell paste was well dispersed in physiological saline, centrifugation at 3000 rpm for 20 minutes was performed, and then supernatant was removed for cell collection. After repeating this 3 times, cells were dispersed in distilled water and freeze-dried to yield dried killed cells of each kind.

Using the dried killed cells prepared in this manner, the IL-12 production inducing activity of the dried killed cells of each kind of lactic acid bacteria was investigated in mouse spleen cells. The spleen of a mouse (BALB/c, female, 12 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in RPMI 1640 culture medium and plated in a volume of 100 µl per well into a 96-well plate. RPMI 1640 culture medium alone or RPMI 1640 culture medium containing 0.2 µg/ml of the above-prepared dried killed cells of lactic acid bacteria dispersed was added in a volume of 100 µl to each well of the above plate, and cultured in an incubator with 5% CO₂ at 37°C for 24 hours and 4 days. After culture, the IL-12 concentration of the culture supernatant was measured by the ELISA method.

The results are shown in Table 1. As the table clearly shows, the lactic acid bacteria belonging to the genus Lactobacillus exhibited IL-12 production inducing activity. Inter alia, Lactobacillus plantarum, especially Lactobacillus plantarum L-137, showed strong activity.

**Table 1**

| Lactic acid bacteria | IL-12 concentration (ng/ml) | |
|---|---|---|
| | 24-hour culture | 4-day culture |
| - | 0.27 | 0.54 |
| Bifidobacterium longum | 0.19 | 0.52 |
| Bifidobacterium bifidum | 0.38 | 0.67 |
| Lactobacillus casei | 0.23 | 0.64 |
| Lactobacillus delbrueckii | 0.39 | 0.73 |
| Lactobacillus acidophilus | 0.49 | 0.96 |
| Lactobacillus plantarum JCM1149 | 0.50 | 0.94 |
| Lactobacillus plantarum L-137 | 3.24 | 5.16 |

### Test example 2: Impact of pH on decline of IL-12 production inducing activity

### Preliminary test

To 200 ml of modified GYP medium, which is a culture medium for lactic acid bacteria, Lactobacillus plantarum L-137 was inoculated in an amount of 1% by weight as a starter, and cultured at 32°C for 24 hours. The pH of the medium was measured at 3, 6, 9, 12, 15, 18 and 24 hours after inoculation.

The results are shown in Table 2. As Table 2 clearly shows, after 18 hours had passed, the pH of the culture medium substantially stopped decreasing.

**Table 2**

| Preliminary test | |
|---|---|
| Culture duration (hr) | pH |
| 0 | 6.8 |
| 3 | 6.5 |
| 6 | 5.7 |
| 9 | 4.7 |
| 12 | 4.2 |
| 15 | 4.0 |
| 18 | 3.9 |
| 24 | 3.8 |

### Main test

To 200 ml of modified GYP medium, which is a culture medium for lactic acid bacteria, Lactobacillus plantarum L-137 was inoculated in an amount of 4% by weight as a starter, and cultured at 32°C for 3, 6, 9, 12, 15, 18 or 24 hours. After each medium was measured for pH, sterilization at 80°C for 20 minutes and subsequent centrifugation at 3000 rpm for 20 minutes were performed. Then supernatant was removed for cell collection. The collected cell paste was well dispersed in physiological saline, centrifugation at 3000 rpm for 20 minutes was performed, and then supernatant was removed for cell collection. After repeating this 3 times, cells were dispersed in distilled water and freeze-dried to yield dried killed cells of each kind.

Using the dried killed cells prepared in this manner, the impact of the culture condition in preparation of the dried killed cells of lactic acid bacteria on the IL-12 production inducing activity in mouse spleen cells was investigated. The spleen of a mouse (BALB/c, female, 29 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate. RPMI 1640 culture medium alone or RPMI 1640 culture medium containing 1 µg/ml of dispersed dried killed cells of Lactobacillus plantarum L-137 cultured for a different period of time was added in a volume of 100 µl to each well of the above plate, and cultured in an incubator with 5% CO₂ at 37°C for 23 hours. After culture, the IL-12 concentration of the culture supernatant was measured by the ELISA method.

The results are shown in Table 3. As Table 3 clearly shows, after 18 hours had passed, continued culture decreased the IL-12 production inducing activity of the dried killed cells of Lactobacillus plantarum L-137. The IL-12 production inducing activity of the dried killed cells obtained in the culture stopped at pH 3.8 was lower by 26% compared with the activity in the case where the culture stopped at pH 4.0. Therefore, it was revealed that in preparation of heat-killed cells of lactic acid bacteria, culture must be stopped at the stage when the pH of the culture medium substantially stops decreasing.

**Table 3**

| Main test | | |
|---|---|---|
| Culture duration (hr) | pH | IL-12 concentration (ng/ml) |
| 15 | 4.0 | 5.4 |
| 18 | 3.9 | 4.9 |
| 21 | 3.9 | 4.6 |
| 24 | 3.8 | 4.0 |

### Test example 3: Decline of IL-12 production inducing activity of lactic acid bacteria left stand after culture

To modified GYP medium, which is a culture medium for lactic acid bacteria, Lactobacillus plantarum L-137 was inoculated in an amount of 1% by weight as a starter, and cultured at 32°C for 18 hours. Immediately after culture, sterilization at 80°C for 20 minutes and subsequent centrifugation at 3000 rpm for 20 minutes were performed. Then supernatant was removed for cell collection. The collected cell paste was well dispersed in physiological saline, centrifugation at 3000 rpm for 20 minutes was performed, and then supernatant was removed for cell collection. After repeating this 3 times, cells were dispersed in distilled water and freeze-dried to yield dried killed cells of Lactobacillus plantarum L-137.

In the same manner, to modified GYP medium, which is a culture medium for lactic acid bacteria, Lactobacillus plantarum L-137 was inoculated in an amount of 1% by weight as a starter, cultured at 32°C for 18 hours, and left stand at 18°C for 7 hours 30 minutes. Then sterilization at 80°C for 20 minutes and subsequent centrifugation at 3000 rpm for 20 minutes were performed. Then supernatant was removed for cell collection. The collected cell paste was well dispersed in physiological saline, centrifugation at 3000 rpm for 20 minutes was performed, and then supernatant was removed for cell collection. After repeating this 3 times, cells were dispersed in distilled water and freeze-dried to yield dried killed cells of Lactobacillus plantarum L-137 left and heated after culture.

Using the dried killed cells prepared in this manner, the impact of the heating condition in preparation of the dried killed cells of lactic acid bacteria on the IL-12 production inducing activity in mouse spleen cells was investigated.

The spleen of a mouse (BALB/c, female, 12 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate. RPMI 1640 culture medium alone or RPMI 1640 culture medium containing 1 µg/ml of dispersed dried killed cells of Lactobacillus plantarum L-137 cultured for a different period of time was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 4. As Table 4 clearly shows, when the culture medium was left stand at a low temperature after culture and then heated, the IL-12 production inducing activity of the obtained dried killed cells decreased. The IL-12 production inducing activity of the dried killed cells left stand after culture and then heated was lower by 37% than that of the dried killed cells heated immediately after culture. Therefore, it was revealed that in preparation of heat-killed cells of Lactobacillus plantarum L-137, heating must be done immediately after culture is stopped. It was also shown that in providing an immunostimulating composition containing Lactobacillus plantarum L -137, the Lactobacillus plantarum L -137 is preferably contained in the form of killed cells since decrease of the IL-12 production inducing activity observed in this test is expected to occur during production processes or storage period if living Lactobacillus plantarum L-137 is used for the production.

**Table 4**

| Dried killed cells used | IL-12 concentration (ng/ml) |
|---|---|
| L-137 dried killed cells heated immediately after culture | 12.6 |
| L-137 dried killed cells left stand after culture and then heated | 8.0 |

### Test example 4: Heat resistance of IL-12 production inducing activity

To modified GYP medium, which is a culture medium for lactic acid bacteria, Lactobacillus plantarum L-137 was inoculated in an amount of 1% by weight as a starter, and cultured at 32°C for 18 hours. Immediately after culture, sterilization at 80°C for 20 minutes and subsequent centrifugation at 3000 rpm for 20 minutes were performed. Then supernatant was removed for cell collection. The collected cell paste was well dispersed in physiological saline, centrifugation at 3000 rpm for 20 minutes was performed, and then supernatant was removed for cell collection. After repeating this 3 times, cells were dispersed in distilled water and freeze-dried to yield dried killed cells of Lactobacillus plantarum L-137.

The dried killed cells of lactic acid bacteria prepared in this manner was dispersed in a concentration of 40% by weight in distilled water, and sterilized at 121°C for 10, 20 or 40 minutes with a high-pressure steam sterilizer. In the same manner, the dried killed cells of lactic acid bacteria dispersed in a concentration of 40% by weight in distilled water was sterilized at 100°C for 10, 20 or 40 minutes.

Using the dried killed cells of lactic acid bacteria dispersed in water and heated, the heat stability of the IL-12 production inducing activity of the dried killed cells of lactic acid bacteria in mouse peritoneal cells was investigated. After peritoneal cells were prepared from a mouse (C57BL/6, female, 16 week old) and counted with an automated blood cell counter, the suspension was adjusted to a concentration of 1.0 × 10⁶ cells/ml in the RPMI 1640 culture medium and plated in a volume of 100 µl per well into a 96-well plate. RPMI 1640 culture medium alone or RPMI 1640 culture medium containing heated Lactobacillus plantarum L-137 equivalent to 0.2 µg/ml of dried killed cells of Lactobacillus plantarum L-137 was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 5. As Table 5 clearly shows, heat treatment of dried killed cells of Lactobacillus plantarum L-137 in aqueous solution at 121°C remarkably decreased the IL-12 production inducing activity. Meanwhile, heat treatment in aqueous solution at 100°C hardly decreased the activity. Consequently, it was shown that immunostimulating effect cannot be expected even if dried killed cells of Lactobacillus plantarum L-137 are added to products such as retort-packed food that passes through a high pressure steam sterilization process at about 110 to 130°C for about 30 to 60 minutes. Meanwhile, under conditions of heat sterilization in producing usual drinks, for example, at 63°C for 30 minutes, at 75°C for 15 seconds or at 120°C for 3 seconds in the production of milk; or at 85°C for 30 minutes in the production of soft drinks of pH 4 or higher, the IL-12 production inducing activity is maintained. As a result, it was shown that, in providing an immunostimulating composition containing lactic acid bacteria, blending into drinks is suitable.

**Table 5**

| Treatment temperature | Treatment duration (min) | IL-12 concentration (ng/ml) |
|---|---|---|
| 121°C | 0 | 1.0 |
| | 10 | 0.6 |
| | 20 | 0.2 |
| | 40 | 0 |
| 100°C | 0 | 1.7 |
| | 10 | 1.7 |
| | 20 | 1.8 |
| | 40 | 1.3 |

### Examples

To modified GYP medium, which is a culture medium for lactic acid bacteria, Lactobacillus plantarum L-137 was inoculated in an amount of 4% by weight as a starter, cultured at 32°C for 24 hours, and sterilized by achieving 80°C. Then, after washing the heat-killed cells with water using a microfiltration membrane, dextrin as much as 4 times the amount of the heat-killed cells was added to the liquid containing the washed cells. By subsequent spray drying, killed cell powder of Lactobacillus plantarum L-137 was obtained. The obtained killed cell powder contains 20% by weight of killed cells of Lactobacillus plantarum L-137. The following food products were prepared using the powder.

### (1) Isotonic drink

After weighing out each ingredient in Table 6, the specified amount of water was added, and the ingredients were dissolved and dispersed adequately. The liquid was sterilized at 98°C for 30 seconds to give a stock solution of a killed-cell-powder-containing isotonic drink and a stock solution of a control isotonic drink. Immediately after preparation, 100 ml of each stock solution was poured into a separate 100 ml transparent bottle, and each bottle was sealed with a polypropylene cap. A killed-cell-powder-containing isotonic drink and a control isotonic drink were thus prepared. Each type of the isotonic drinks thus prepared was stored at 4°C and 40°C for 2 weeks, and the IL-12 production inducing activity of the contained killed cell powder was investigated.

**Table 6**

| Killed-cell-powder-containing isotonic drink | | Control isotonic drink | |
|---|---|---|---|
| Ingredient | Amount (g) | Ingredient | Amount (g) |
| Fructose | 39 | Fructose | 39 |
| Reduced maltose | 8 | Reduced maltose | 8 |
| Lemon juice | 10 | Lemon juice | 10 |
| Vitamin C | 2 | Vitamin C | 2 |
| Flavor | 1 | Flavor | 1 |
| Citric acid | 0.5 | Citric acid | 0.5 |
| Killed cell powder | 0.5 | Killed cell powder | - |
| Water | q. s. to 1000 ml in total | Water | q. s. to 1000 ml in total |

The spleen of a mouse (BALB/c, female, 7 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate.

The killed-cell-powder-containing isotonic drink stored at 4°C for 2 weeks and then diluted 1000-fold with RPMI 1640 culture medium (containing 500 ng/ml of killed cell powder of Lactobacillus plantarum L-137), the killed-cell-powder-containing isotonic drink stored at 40°C for 2 weeks and then diluted 1000-fold with RPMI 1640 culture medium, the control isotonic drink stored at 4°C for 2 weeks and then diluted 1000-fold with RPMI1640 culture medium, RPMI 1640 culture medium alone or RPMI 1640 culture medium containing 500 ng/ml of dispersed killed cell powder of Lactobacillus plantarum L-137 was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 7. As Table 7 clearly shows, even after preparation of the isotonic drink, the IL-12 production inducing activity of the killed cell powder of Lactobacillus plantarum L-137 was maintained. Also, even under the storage condition of 40°C, where quality deterioration is accelerated, the decrease of the IL-12 production inducing activity was slight. From these results, it was shown that the killed cell powder of Lactobacillus plantarum L-137 can be blended into isotonic drinks which are produced under usual sterilization conditions and distributed at room temperature, without loss of its activity.

**Table 7**

| Sample material | IL-12 concentration (ng/ml) |
|---|---|
| RPMI 1640 culture medium | 0.5 |
| Control isotonic drink | 0.6 |
| Killed cell powder | 4.9 |
| Killed-cell-powder-containing isotonic drink stored at 4°C for 2 weeks | 5.5 |
| Killed-cell-powder-containing isotonic drink stored at 40°C for 2 weeks | 4.0 |

### (2) Fruit drink

After weighing out each ingredient in Table 8, the specified amount of water was added, and the ingredients were dissolved and dispersed adequately. The liquid was sterilized at 98°C for 30 seconds to give a stock solution of a killed-cell-powder-containing fruit drink and a stock solution of a control fruit drink. Immediately after preparation, 100 ml of each stock solution was poured into a separate 100 ml transparent bottle, and each bottle was sealed with a polypropylene cap. A killed-cell-powder-containing fruit drink and a control fruit drink were thus prepared. Each type of the fruit drinks thus prepared was stored at 4°C and 40°C for 2 weeks, and the IL-12 production inducing activity of the contained killed cell powder was investigated.

**Table 8**

| Killed-cell-powder-containing fruit drink | | Control fruit drink | |
|---|---|---|---|
| Ingredient | Amount (g) | Ingredient | Amount (g) |
| High-fructose corn syrup | 128 | High-fructose corn syrup | 128 |
| Orange juice | 110 | Orange juice | 110 |
| Granulated sugar | 5 | Granulated sugar | 5 |
| Citric acid | 3 | Citric acid | 3 |
| Vitamin C | 1 | Vitamin C | 1 |
| Flavor | 1 | Flavor | 1 |
| Killed cell powder | 0.5 | Killed cell powder | - |
| Water | q. s. to 1000 ml in total | Water | q. s. to 1000 ml in total |

The spleen of a mouse (BALB/c, female, 7 week old) was removed, crushed in RPMI 1640 culture medium, and filtered through a #200 mesh filter to give a spleen cell suspension.

After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate.

The killed-cell-powder-containing fruit drink stored at 4°C for 2 weeks and then diluted 1000-fold with RPMI 1640 culture medium (containing 500 ng/ml of killed cell powder of Lactobacillus plantarum L-137), the killed-cell-powder-containing fruit drink stored at 40°C for 2 weeks and then diluted 1000-fold with RPMI 1640 culture medium, the control fruit drink stored at 4°C for 2 weeks and then diluted 1000-fold with RPMI 1640 culture medium, RPMI 1640 culture medium alone or RPMI 1640 culture medium containing 500 ng/ml of dispersed dried killed cell powder of Lactobacillus plantarum L-137 was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 9. As Table 9 clearly shows, in the fruit drink stored at 4°C after preparation, the IL-12 production inducing activity of the killed cell powder of Lactobacillus plantarum L-137 was maintained. Meanwhile, under the storage condition of 40°C, where quality deterioration is accelerated, the decrease of the IL-12 production inducing activity was significant. From these results, it was shown that the killed cell powder of Lactobacillus plantarum L-137 can be blended into fruit drinks which are produced under usual sterilization conditions and distributed at chilled temperature, without loss of its activity.

**Table 9**

| Sample material | IL-12 concentration (ng/ml) |
|---|---|
| RPMI 1640 culture medium | 0.6 |
| Control fruit drink | 0.6 |
| Killed cell powder | 4.4 |
| Killed-cell-powder-containing fruit drink stored at 4°C for 2 weeks | 3.9 |
| Killed-cell-powder-containing fruit drink stored at 40°C for 2 weeks | 1.4 |

### (3) Tofu

Processed soybean and killed cell powder in the respective amounts shown in Table 10 were added into the specified amount of water, dispersed adequately, and boiled at low heat for 4 minutes. Heating was stopped, coagulant was added, and vigorous stirring for 10 seconds was performed. Immediately after that, the liquid was poured into a 100 ml aluminum foil pouch with a spout, sterilized at 75°C for 5 minutes, and left until cooled to give a killed-cell-powder-containing tofu and a control tofu. The IL-12 production inducing activity of thus-obtained tofu preparations was investigated.

**Table 10**

| Killed-cell-powder-containing tofu | | Control tofu | |
|---|---|---|---|
| Ingredient | Amount (g) | Ingredient | Amount (g) |
| Processed soybean* | 116 | Processed soybean* | 116 |
| Water | 878.5 | Water | 878.5 |
| Coagulant* | 5 | Coagulant* | 5 |
| Killed cell powder | 0.5 | Killed cell powder | - |

| | | | |
|---|---|---|---|
| *Hon-tofu (Tofu mix by House Foods Corp.) | | | |

The spleen of a mouse (BALB/c, female, 20 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate.

The killed-cell-powder-containing tofu adequately dispersed and diluted 1000-fold with RPMI 1640 culture medium (containing 500 ng/ml of killed cell powder of Lactobacillus plantarum L-137), the control tofu adequately dispersed and diluted 1000-fold with RPMI 1640 culture medium, mixture of 50 µl of the control tofu adequately dispersed and diluted 500-fold with RPMI 1640 culture medium and 50 µl of RPMI 1640 culture medium containing dispersed dried killed cell powder of Lactobacillus plantarum L-137 in a concentration of 1000 ng/ml, or RPMI 1640 culture medium alone was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 11. As Table 11 clearly shows, between the cases where the killed cell powder was added at the time of tofu preparation and where the powder was added just before measurement, no significant difference of IL-12 production inducing activity was observed. From these results, it was shown that the killed cell powder of Lactobacillus plantarum L-137 can be blended into tofu produced in a usual process, without loss of its activity.

**Table 11**

| Sample material | IL-12 concentration (ng/ml) |
|---|---|
| RPMI 1640 culture medium | 0.6 |
| Control tofu | 0.8 |
| Control tofu + killed cell powder | 3.3 |
| Killed-cell-powder-containing tofu | 2.7 |

### (4) Chocolate

Chocolate in the amount shown in Table 12 was crushed, the specified amount of killed cell powder and dextrin were added thereto and dispersed, and the mixture was kneaded at 30°C for 10 minutes. The kneaded chocolate was poured into a mold and then left until cooled to give a killed-cell-powder-containing chocolate and a control chocolate. The IL-12 production inducing activity of thus-obtained chocolate preparations was investigated.

**Table 12**

| Killed-cell-powder-containing chocolate | | Control chocolate | |
|---|---|---|---|
| Ingredient | Amount (g) | Ingredient | Amount (g) |
| Chocolate* | 989 | Chocolate* | 989 |
| Dextrin | 10 | Dextrin | 10 |
| Killed cell powder | 1 | Killed cell powder | - |

| | | | |
|---|---|---|---|
| *Ingredient for homemade sweets (by K's Factory) | | | |

The spleen of a mouse (BALB/c, female, 7 week old) was removed, crushed in RPMI 1640 culture medium, and filtered through a #200 mesh filter to give a spleen cell suspension. After the cells in the spleen cell suspension were counted with an automated blood cell counter, the suspension was adjusted to a concentration of 5 × 10⁶ cells/ml in the RPMI 1640 culture medium and plated in a volume of 100 µl per well into a 96-well plate.

The killed-cell-powder-containing chocolate melted and then adequately dispersed and diluted 2000-fold with RPMI 1640 culture medium (containing 500 ng/ml of killed cell powder of Lactobacillus plantarum L-137), the control chocolate adequately dispersed and diluted 2000-fold with RPMI 1640 culture medium, mixture of 50 µl of the control chocolate melted and then adequately dispersed and diluted 1000-fold with RPMI 1640 culture medium and 50 µl of RPMI 1640 culture medium containing dispersed dried killed cell powder of Lactobacillus plantarum L-137 in a concentration of 1000 ng/ml, or RPMI 1640 culture medium alone was added in a volume of 100 µl to each well of the above plate, and cultured in an incubator with 5% CO₂ at 37°C for 24 hours. After culture, the IL-12 concentration of the culture supernatant was measured by the ELISA method.

The results are shown in Table 13. As Table 13 clearly shows, between the cases where the killed cell powder was added at the time of chocolate preparation and where the powder was added just before measurement, no difference of IL-12 production inducing activity was observed. From these results, it was shown that the killed cell powder of Lactobacillus plantarum L-137 can be blended into chocolate produced in a usual process, without loss of its activity.

**Table 13**

| Sample material | IL-12 concentration (ng/ml) |
|---|---|
| RPMI 1640 culture medium | 0.5 |
| Control chocolate | 0.6 |
| Control chocolate + killed cell powder | 1.1 |
| Killed-cell-powder-containing chocolate | 1.1 |

From these results, it was shown that the IL-12 production inducing activity of the isotonic drink, fruit drink, tofu and chocolate containing killed cell powder of Lactobacillus plantarum L-137 was higher than that of controls not containing the powder. The above Examples showed that the IL-12 production inducing activity of killed cell powder of Lactobacillus plantarum L-137 was maintained even when high-water-content food and high-fat food, of which quality maintenance is relatively difficult, were prepared and/or stored. Consequently, it is obvious that the IL-12 production inducing activity of killed cell powder of Lactobacillus plantarum L-137 can be maintained when dried food, of which quality maintenance is relatively easy, is prepared and stored. That is, by adding lactic acid bacteria belonging to the genus Lactobacillus to a drink, a fermented food, a semisolid food, a solid food, a powdered food, IL-12 production can be effectively induced in the body. Consequently, by regularly taking such a food-or-drink product, health promotion can be realized.

## Claims

1. A method for producing an immunostimulating composition containing lactic acid bacteria having IL-12 production-inducing activity, comprising culturing the lactic acid bacteria and then killing the lactic acid bacteria within 30 minutes from the point of time at which the decrease of the pH of the culture medium gets less than 0.1 per 3 hours, and adding the killed cells of the lactic acid bacteria to a food-or-drink product, wherein the lactic acid bacteria are Lactobacillus plantarum L-137.

2. The method according to claim 1, wherein the lactic acid bacteria are killed by heat.

3. The method of claim 1 or 2, wherein the food-or-drink product is a drink.

4. The method according to claim 3, wherein the drink is selected from the group consisting of energy drink, sports supplement drink, isotonic drink, functional water,
bottled water, flavored water, flavored drink, milk-free meal-substitute drink, fruit juice, fruit drink, vegetable juice, coffee drink, tea drink, green tea drink, oolong tea drink and blended tea-based drink.

5. The method of claim 1 or 2, wherein the food-or-drink product is a powdered food.

6. The method according to claim 5, wherein the powdered food is selected from the group consisting of powdered drink, powdered energy drink, powdered sports supplement drink, powdered isotonic drink, powdered functional water, powdered bottled water, powdered flavored water, powdered flavored drink, powdered cocoa, powdered malt drink, powdered soup, tabletop artificial sweetener, powdered tea, powdered green tea, powdered plum tea, powdered seaweed tea, powdered juice and instant coffee.

## Patentansprüche

1. Verfahren zur Herstellung einer immunstimulierenden Zusammensetzung, enthaltend Milchsäurebakterien mit die IL-12-Produktion induzierender Aktivität, umfassend das Kultivieren der Milchsäurebakterien und dann Abtöten der Milchsäurebakterien innerhalb von 30 Minuten ab dem Zeitpunkt, wo die Verringerung des pH-Wertes des Kulturmediums weniger wird als 0,1 pro 3 Stunden, und Zugeben der abgetöteten Zellen der Milchsäurebakterien zu einem Nahrungsmittel- oder Getränkeprodukt, wobei die Milchsäurebakterien Lactobacillus plantarum L-137 sind.

2. Verfahren nach Anspruch 1, wobei die Milchsäurebakterien mit Wärme abgetötet werden.

3. Verfahren nach Anspruch 1 oder 2, wobei das Nahrungsmittel- oder Getränkeprodukt ein Getränk ist.

4. Verfahren nach Anspruch 3, wobei das Getränk ausgewählt ist aus der Gruppe, bestehend aus einem Energydrink, einem Sportergänzungsgetränk, einem isotonischen Getränk, funktionellem Wasser, Flaschenwasser, Wasser mit Geschmack, einem aromatisierten Getränk, einem milchfreien Mahlzeitersatzgetränk, Fruchtsaft, einem Fruchtgetränk, Gemüsesaft, einem Kaffeegetränk, einem Teegetränk, einem Grünteegetränk, einem Oolongteegetränk und einem Mischtee-basierten Getränk.

5. Verfahren nach Anspruch 1 oder 2, wobei das Nahrungsmittel- oder Getränkeprodukt ein Nahrungsmittelpulver ist.

6. Verfahren nach Anspruch 5, wobei das Nahrungsmittelpulver ausgewählt ist aus der Gruppe, bestehend aus Getränkepulver, Energydrink-Pulver, Sportergänzungsgetränkepulver, isotonischem Getränkepulver, Pulver für funktionelles Wasser, Pulver für Flaschenwasser, Geschmackspulver für Wasser, aromatisiertem Getränkepulver, Kakaopulver, Malzgetränkepulver, Suppenpulver, Tafel-Süßstoff, Teepulver, Grünteepulver, Pflaumenteepulver, Meeresalgenteepulver, Saftpulver und Instantkaffee.

## Revendications

1. Une méthode pour produire une composition immunostimulante contenant des bactéries d'acide lactique présentant une activité induisant la production d'IL-12, comprenant une mise en culture des bactéries d'acide lactique et ensuite une destruction desdites bactéries d'acide lactique endéans 30 minutes à partir du moment où la diminution de pH du milieu de culture est inférieur à 0.1 par 3 heures, et une addition des cellules détruites desdites bactéries d'acide lactique à un produit alimentaire ou une boisson, dans laquelle les bactéries d'acide lactique sont des Lactobacillus plantarum L-137.

2. Méthode selon la revendication 1, dans laquelle les bactéries d'acide lactique sont détruites par chaleur.

3. Méthode selon la revendication 1 ou 2, dans laquelle le produit alimentaire ou la boisson est une boisson.

4. Méthode selon la revendication 3, dans laquelle la boisson est choisie dans le groupe constitué de boisson énergétique, boisson complémentaire pour sportifs, boisson isotonique, eau fonctionnelle, boisson en bouteille, eau aromatisée, boisson aromatisée, boisson de substitut de repas exempt de lait, jus de fruit, boisson fruitée, jus de légumes, boisson à base de café, boisson à base de thé, boisson à base de thé vert, boisson à base de thé Oolong et boisson à base d'un mélange de thé.

5. Méthode selon la revendication 1 ou 2, dans laquelle le produit alimentaire ou la boisson est un aliment en poudre.

6. Méthode selon la revendication 5, dans laquelle l'aliment en poudre est choisi dans le groupe constitué de boisson en poudre, boisson énergétique en poudre, boisson complémentaire en poudre pour sportifs, boisson isotonique en poudre, eau fonctionnelle en poudre, eau pulvérulente en bouteille, eau aromatisée sous forme de poudre, boisson aromatisée sous forme de poudre, cacao en poudre, boisson à base de malte en poudre, soupe en poudre, édulcorant artificiel de table, thé en poudre, thé vert en poudre, thé aux prunes en poudre, thé à base d'algues en poudre, jus en poudre et café instantané.
